# EUROPEAN PATENT SPECIFICATION

(11) **EP 3 878 430 B1**
(45) Date of publication and mention of the grant of the patent: **25.06.2025**
(21) Application number: 21161169.4
(22) Date of filing: 08.03.2021
(51) Int. Cl.: A61K 8/06, A61K 8/23, A61K 8/34, A61K 8/36, A61K 8/67, A61K 8/68, A61Q 19/08, A61K 9/00

(54) **TOPICAL ANTI-AGING SELF-EMULSIFYING COMPOSITION**
TOPISCHE ANTI-FALTEN SELBSTEMULGIERENDE ZUSAMMENSETZUNG
COMPOSITION TOPIQUE AUTO-ÉMULSIFIANTE ANTI-VIEILLISSEMENT

(30) Priority: 11.03.2020 IT 202000005224
(43) Date of publication of application: 15.09.2021
(73) Proprietor: Mitochon S.r.l., 40068 San Lazzaro di Savena (BO) (IT)
(72) Inventor: FRATTER, Andrea, 30030 Olmo di Martellago (VE) (IT); BUSCAROLI, Silvia, 40068 San Lazzaro di Savena (BO) (IT); FEDERICI, Ettore, 40068 San Lazzaro di Savena (BO) (IT)
(74) Representative: Perani & Partners S.p.A.

(56) References cited:
- DATABASE GNPD [online] MINTEL; 16 December 2011 (2011-12-16), ANONYMOUS: "Eternel Handful of Integral Youth", XP055752275, retrieved from www.gnpd.com Database accession no. 1694448
- "Profil Integral Correction Care Neck & Décolleté", GNPD, 1 December 2011 (2011-12-01), XP002735515

## Description

### FIELD OF THE INVENTION

The present invention relates to a topical composition comprising an oily phase comprising active ingredients with antioxidant action, based on coenzyme Q10 and glutathione, and an aqueous phase capable of generating a self-emulsion promoting the absorption thereof at the skin tissue level. Such a composition can be used for cosmetic purposes for treating and preventing so-called photo-chrono skin aging and for improving dermal trophism.

A further object of the present invention is the method for preparing the aforementioned topical composition.

### STATE OF THE ART

The phenomenon of cellular aging is a natural process of decay of cellular physiological functions which can sometimes be induced or accelerated by external causes, such as solar radiation. In this specific case, reference is made to photo-aging. Visually, the phenomenon manifests itself with the appearance at the skin level, especially for photo-exposed areas such as the face, neck and trunk, of more or less numerous and deep wrinkles, more or less widespread menalistic spots, epidermal thickening, sunspots and lentigo. Cellular photo-aging has a complex nature from a cellular biochemical perspective and can involve the deeper layers of the skin. Sometimes, clinical findings typical of skin photo-aging are followed by or associated with lesions of a more serious nature with skin cells which manifest themselves in the form of neoplastic lesions (squamous and basal cell carcinomas, melanomas) and preneoplastic lesions (actinic keratoses). The main solar radiation involved in the photo-aging phenomenon is of the ultraviolet type A (UV-A) and/or type B (UV-B) and infrared radiation type A (or IRA). While the UV-B radiation is absorbed at the superficial level of the epidermis, UV-A and IRA can penetrate deeper: the former at the dermal level and the latter predominantly at the subcutaneous tissue level. Specifically, solar radiation can induce an alteration of finely regulated cellular physiological mechanisms, particularly at the cellular mitochondria level. Mitochondria are small organelles present in eukaryotic cells which are responsible for cell respiration processes and endowed with their own genetic material (mtDNA). In particular, cellular respiration is a multi-phase process for the production of energy in the form of ATP (adenosine triphosphate) by the demolition of complex molecules to simpler molecules. The last phase of cellular respiration is oxidative phosphorylation. In detail, there are five protein complexes located at the level of the internal mitochondrial membrane, which generate an electrochemical proton gradient through electron chain translocation, coming from previous metabolic processes. Such electrons react with molecular oxygen to ultimately promote the chemical reaction which ATP produces from ADP (adenosine diphosphate). During this last electronic step, it is naturally possible to form reactive oxygen species (or ROS). Reactive oxygen species are reactive and therefore unstable radical molecules since they seek to restore the electrical balance thereof by reacting with nucleophilic molecules present in tissues. This results in the formation of further cascading radical reactions and additional reactive radical species resulting in molecular destruction and loss of the biological function of cellular structures. Examples of nucleophilic molecules in our body are lipids, proteins, and genetic material (DNA or RNA) essential for cell structure and function. For example, reacting with ROS, the unsaturated lipids of cell membranes lose the function thereof of maintaining the fluidity and plasticity of the cell membranes themselves; further, the reaction of ROS with proteins induces a modification of the three-dimensional or quaternary structure of the latter, altering the biological function thereof. Finally, reacting with ROS, the nitrogenous DNA bases can also undergo mutation phenomena which, if not readily corrected by endogenous gene repair systems, cause processes of cellular apoptosis (so-called cellular suicide) or the triggering of neoplastic processes. Indeed, mutations at the DNA level can inhibit the expression of antioncogenes, such as the antioncogene p53. The molecular mechanisms resulting from the action of solar radiation have recently been outlined with the scientific "defective powerhouse" theory (1).

On the other hand, although ROS formation is a process which, as mentioned, occurs naturally in the cellular environment and especially in the mitochondrial one, it could be amplified by the solar radiation itself. This results in alterations of the mitochondrial DNA of the cutaneous fibroblasts as well as of the cells responsible for the synthesis/demolition regulation of collagen proteins and the extracellular dermal matrix. Mitochondrial DNA is generally more sensitive than nuclear DNA because it lacks transcriptional regulatory protein structures, such as histones. Said gene mutations directly result in an alternation and reduction of the entire mitochondrial function. This specifically results in an increase in the production of catabolic enzymes, such as collagenases, gelatinases or various proteases and, more specifically, of the collagen degradation enzyme and the enzyme matrix-metalloproteinase 1 (or MMP-1). At the same time, there is a proportional reduction in the synthesis of anabolic enzymes in general, and specifically of the metalloproteinase 1 inhibitor MMP-1 (or TIMP-1). Further, a single exposure to solar radiation may result in a reduction in the expression of genes involved in *de novo* collagen synthesis, such as COL1A1. The alteration of the extracellular matrix consists in the deficiency of glucosaminoglycans and, consequently, of proteoglycans responsible for dermal hydration. Therefore, overall, the balance of regulation shifts towards catabolic activity and a slow and progressive alteration of the dermal tissue which is therefore less compact, less hydrated and above all less elastic. Furthermore, even in the absence of exposure to solar radiation, mitochondrial DNA-induced UV mutations have been observed to persist. This can be explained by considering that a now defective mitochondrial respiratory chain leads to increased ROS production, which in turn, like in a vicious circle, induces further mutagenesis, regardless of the inducing agent. The chronization of this complex cellular mechanism induces a reduction in collagen fibres over time and a generous increase in collagen degradation fragments with relative visual signs of skin aging. In addition, both UV-B and UV-A rays are responsible for the occurrence of skin cancers (squamous and basal cell carcinomas and melanomas); in particular for melanomas, the epidemiology seems to have established the link between excessive sun exposure in childhood and adolescence and increased risk of developing melanomas in adulthood.

Another phenomenon related to cellular aging is the development of so-called AGEs ("Advanced Glycation End products") as well as products of the glycation reaction of lipids, proteins and/or nucleotides by exposure to sugars and the accumulation thereof contributes to oxidative stress and to triggering inflammatory or immune-mediated processes. "Glucosepane" is an example of AGE, i.e., a cross-linked lysine-arginine product resulting from the chemical reaction between the D-glucose aldose sugar present in tissues and the nucleophilic groups of proteins, mainly amine. Specifically, the chemical reaction generates a Schiff base through the Maillard reaction. Such glycation phenomena are particularly significant in light of the chemical modifications which they can cause to dermal-epidermal structural proteins, including collagen, causing cross-linking phenomena and relative loss of functionality and accelerated turnover.

In order to counteract such phenomena responsible for the slow and continuous alteration of skin tissues and damage to cellular gene material, lifestyles should be adopted which are best suited to limiting contact with external agents responsible for such alterations (primarily sunlight and UV sources) as much as possible or at least reducing the harmful effects thereof on the skin with the use of adequate protective devices (UV filters) and nutritional supplementation aids containing substances capable of counteracting the development of oxygen free radicals at the cellular level and preventing the formation of AGEs.

Fortunately, endogenous substances of peptide and isoprene nature exist, such as reduced glutathione tripeptide (or GSH) and oxidized coenzyme Q10 (CoQ10 or ubiqinone), respectively, which constitute the fundamental antioxidant agents in mitochondrial radical homoeostasis. In particular, GSH glutathione is a cofactor of the enzyme glutathione peroxidase with "scavenger" action of ROS. Molecules with free thiol groups, such as lipoic acid, N-acetylcysteine (or NAC) and the same GSH glutathione are capable of penetrating both the aqueous compartment of the cell and the lipid membrane compartment, helping to neutralize the excessive generation of ROS, in particular peroxides and superoxides, both enzymatically (regeneration of GSH peroxidase) and oxidoreductively, reducing the mitochondrial damage. On the other hand, coenzyme Q10 is an electron transporter in the mitochondrial respiratory chain. Depending on the oxidation state, it can be present in three forms: an oxidized form, an intermediate semi-quinone, and a reduced form.

The most direct route to prevent oxidative damage which preludes the development of skin aging and connective tissue degradation is topical, through emulsion or gel formulations. The topical route avoids the metabolization of the active substances conveyed, such as GSH or coenzyme Q10, by the intestine and liver since such metabolization would reduce the tissue concentrations of the substances to values too low to ensure the efficacy thereof. On the other hand, the stratum corneum, composed of corneocytes and cementing intercellular substance, which in turn consists of lipids such as cholesterol, ceramides and saturated fatty acids ("brick and lime model"), represents the main biomechanical obstacle to the permeation of substances conveyed through the epidermis. In addition to this, another problem related to the skin is the concentration gradient which occurs between the skin surface and the sub-corneal areas. Finally, it is necessary to consider the process of degradation of substances which, due to the high reactivity thereof, can reach the cell area already in oxidized form or no longer functional due to the presence of enzymes, such as transaminases or hydrolases. Overall, therefore, through the topical route it is often not possible to ensure that substances reach the cellular and subcellular target thereof intact and in adequate concentrations in the most distal tissues, such as the dermal connective tissue. For these reasons it is most definitely necessary to provide a technological carrier which allows, on the one hand, the effective permeation of the active substances through the intact skin and, on the other hand, the protection thereof against oxidative and degrading phenomena.

*Eternel Handful of Integral Youth* (XP055752275) is a commercial product which comprises the actives glutathione and coenzyme Q10, beyond other ingredients, such as: water, cetearyl alcohol, ascorbyl palmitate, tocopheryl acetate, caprylic/capric triglyceride, cholesterol, oleate, ions such as chloride, sodium, magnesium, and sulphate.

*Profil Integral Correction Care Neck & Décolleté* (XP002735515) regards a commercial product comprising GSH, CoQ10, Ethylhexyl palmitate beyond other ingredients.

### SUMMARY OF THE INVENTION

The applicant has now surprisingly found that such an innovative technological carrier can be achieved by the present invention. The subject of the invention is in fact a topical composition comprising as active ingredients coenzyme Q10 in oxidized form and glutathione in reduced form comprising:
A) an oily phase in which the aforementioned active ingredients are dispersed and comprising:
   A1) oleic acid,
   A2) ascorbyl palmitate and
   A3) at least one of the following components promoting the absorption of the mixture at the skin tissue level selected from cetearyl alcohol, glyceryl stearate, sorbitan stearate, cetearyl glucoside, potassium cetyl phosphate, tocopheryl acetate.
B) an aqueous phase in which at least one osmotically active electrolytic agent, selected from magnesium sulphate, magnesium chloride, sodium chloride, is dissolved,
wherein the oily phase A) contains oleic acid A1) in quantities comprised between 20% and 50% by weight on the total weight of the composition and ascorbyl palmitate A2) in molar ratio 1:1 with the oxidized form of coenzyme Q10.

### DETAILED DESCRIPTION

For the purposes of the present invention, the term "comprising" or "containing" is not intended to exclude the presence of further components not listed after such a definition, while the term "consisting" or "constituted" is meant to exclude further components not listed after such a definition.

For the purposes of the present invention, the term "active ingredient" means a substance having biological activity.

For the purposes of the present invention, an osmotically active electrolytic agent is intended as a substance which dissociates in aqueous solution into ions and is thereby capable of promoting the osmotic passage of water.

In the topical composition subject of the present invention, the oily phase A) contains at least two, preferably at least three, more preferably at least four of the components A3).

In a more preferred topical composition the oily phase A) contains at least five, preferably all six components A3).

The lipophilic component of the oily carrier (or oily phase or even oil-dispersed system) is capable of promoting permeation at the skin level, especially at the level of the stratum corneum.

In the topical composition according to the present invention, preferably the coenzyme Q10 is present in quantities comprised between 0.10% and 10% by weight on the total weight of the composition and the glutathione in reduced form is in quantities comprised between 0.10% and 10% by weight on the total weight of the composition. The oily phase A) contains oleic acid A1) in quantities comprised between 20% and 50% by weight on the total weight of the composition and ascorbyl palmitate A2) in molar ratio 1:1 with the oxidized form of coenzyme Q10.

According to a preferred embodiment, in the topical composition of the invention the concentration of each of at least one of the components A3) is comprised between 0.10% and 10% by weight on the total weight of the composition while when the topical composition comprises all six components the total amount of said components A3) is ≤ 60% by weight on the total weight of the composition.

Preferably in said topical composition the water is in quantities comprised between 5.0% and 10% by weight on the total weight of the composition.

Preferably in said topical composition the aqueous phase contains at least one osmotically active electrolytic agent in a total concentration comprised between 5.0% and 20% by weight on the total weight of said aqueous phase.

Thus, the topical composition, by virtue of the fact that it comprises an oily phase (or oily carrier or even oil-dispersed system) and an aqueous phase, is capable of generating a self-emulsion.

Without wishing to be bound by any scientific theory, it is held that the oil-dispersed system (or oily phase or oily carrier) generates a self-emulsifying micellar complex capable of acting as a promoter of skin absorption and at the same time of preventing oxidative and degradative phenomena by virtue of the coenzyme Q10 and GSH. Specifically, the mechanism which would lead to the formation of said liquid-crystalline type self-emulsion is based on the following two events, which would occur in parallel:
- partial ionization of the oleic acid and ascorbyl palmitate, after hydrolysis of the latter, once dislocated in the epidermis; these acidic substances tend to ionize once in contact with water present at tissue level with a pH close to neutrality and, specifically, comprised between 7.0 < pH < 7.4;
- the drawing of tissue water into the micellar system induced by the osmotic force of the dispersed osmotically active electrolytic agent.

Such a self-emulsifying system is characterized by high elasticity and is capable of preventing degradation processes for the active ingredient reduced glutathione or GSH (such as enzymatic transamination mechanisms) in addition to favouring the dislocation of coenzyme Q10 in reduced form (ubiquinol).

In a preferred embodiment, the oily phase of said topical composition comprises at least one further active ingredient with depigmenting agents, moisturizing agents, sun filters and/or eutrophic agents preferably selected among: N-acetylglucosamine, retinyl palmitate, NP ceramide, AP ceramide, EOP ceramide, phytofingosine, cholesterol, ethylhexyl methoxycinnamate, octocrylene, avobenzone and combinations thereof.

Depigmenting action is intended as the action of functional substances capable of blocking or inhibiting melanin synthesis.

Moisturizing action is intended as the action of increasing the concentration of water at tissue level.

For the purposes of the present invention, said "sun filters" are chemicals capable of absorbing solar radiation through an action mechanism which could be defined as analogous to that exerted by melanin, the natural pigment responsible for tanning. Eutrophic agents are intended as substances capable of nourishing the skin tissue areas of the body and promoting the general well-being thereof.

The topical composition of the invention can be directly applied to the skin or, prior to being applied to the skin, can in turn be dispersed in oil in water or water in oil emulsions, gels, creams, ointments.

A further object of the present invention is the method for preparing said topical composition comprising the steps of:
i) melting in succession, preferably in a stainless steel capsule, at least one of the components A3) selected from cetearyl alcohol, glycerl stearate, sorbitan stearate, cetearyl glucoside, potassium cetyl phosphate, tocopherol acetate and oleic acid A1) at 60°C under stirring until complete and homogeneous dissolution to obtain an oily phase consisting of a translucent liquid without aggregates;
ii) dissolving the ascorbyl palmitate A2) in said oily phase i) under mechanical stirring (preferably up to 200 rpm) until the complete and homogeneous dissolution thereof while maintaining a constant temperature at 60°C;
iii) combining coenzyme Q10 with said oily phase ii) at the same temperature;
iv) cooling to 50°C and adding to said oily phase iii) glutathione in a reduced form until a suspension is obtained;
v) heating the aqueous phase to 50°C and dissolving the osmotically active electrolyte agent, preferably magnesium sulphate anhydrous;
vi) joining the aqueous phase v) to the oily phase iv) at 50°C under stirring for 5 minutes;
vii) cooling to room temperature and adding optionally at least one of the other active substances with depigmenting agents, moisturizing agents, sun filters and eutrophic agents mentioned above.
viii) homogenizing the mixture vii) preferably for 5 minutes at ¾ of the total power.

Said topical composition can preferably be used in the cosmetic field and more preferably in the treatment and prevention of photo-chrono skin aging and to improve dermal trophism.

By way of non-limiting illustration, an example of said topical composition is given below.

### Example of topical composition with anti-aging action

| ***INCI*** | **Quantity x 100 g** |
|---|---|
| Cetearyl alcohol (and) glyceryl stearate (and) sorbitan stearate (e) cetearyl Glucoside | 35.0000 |
| Ascorbyl palmitate | 0.85000 |
| Tocopheryl acetate | 0.40000 |
| Caprylic/capric triglyceride | 10.00000 |
| Cholesterol | 0.20000 |
| Retinyl palmitate | 0.40000 |
| Octyldodecanol | as needed 100 gr |
| Potassium cetyl phosphate | 0.20000 |
| Cetyl ethylhexanoate | 12.00000 |
| Ethylhexyl palmitate | 12.00000 |
| Dibutyl adipate | 8.00000 |
| Avobenzone | 2.50000 |
| Octocrylene | 2.50000 |
| Ubidecarenone | 0.41000 |
| Oleic acid | 1.00000 |
| Water | 5.00000 |
| Magnesium Sulphate | 0.25000 |
| Glutathione | 0.40000 |
| NP Ceramide; AP Ceramide; EOP Ceramide; Phytosfingosine; Cholesterol; Sodium Lauroyl Lactylate; Carbomer; Xanthan gum | 0.40000 |
| Perfume | 0.30000 |
| | ***100.00000*** |

### REFERENCES

*(1)* Krutmann J, Schroeder P., J. Investig. Dermatol. Symp. Proc., 2009 Aug., 14 (1), 44-9.

## Claims

1. Topical composition comprising coenzyme Q10 in oxidized form and glutathione in reduced form as active ingredients comprising:
A) an oily phase in which the above mentioned active ingredients are dispersed and comprising:
A1) oleic acid,
A2) ascorbyl palmitate and
A3) at least one of the following components promoting the absorption of the mixture at the skin tissue level selected from cetearyl alcohol, glyceryl stearate, sorbitan stearate, cetearyl glucoside, potassium cetyl phosphate, tocopheryl acetate;
B) an aqueous phase in which at least one osmotically active electrolytic agent, selected from magnesium sulphate, magnesium chloride, sodium chloride, is dissolved,
wherein the oily phase A) contains oleic acid A1) in quantities comprised between 20% and 50% by weight on the total weight of the composition and ascorbyl palmitate A2) in molar ratio 1:1 with the oxidized form of coenzyme Q10.

2. Topical composition according to claim 1, wherein the oily phase A) contains at least two, preferably at least three, more preferably at least four components A3).

3. Topical composition according to claim 1, where the oily phase A) contains at least five , preferably all the six components A3).

4. Topical composition according to anyone of claims 1-3, wherein the oily phase A) contains coenzyme Q10 in quantities comprised between 0.10% and 10% by weight on the total weight of the composition and glutathione in reduced form in quantities comprised between 0.10% and 10% by weight on the total weight of the composition.

5. Topical composition according to anyone of claims 1-4, wherein the concentration of each of at least one of the components A3) is comprised between 0,10% and 10% by weight of the total weight of the composition and the total quantity of said components A3) is ≤60% by weight on the total weight of the composition, when all six components A3) are present.

6. Topical composition according to anyone of claims 1-5, wherein the aqueous phase is present in quantities comprised between 5.0% and 10% by weight on the total weight of the composition.

7. Topical composition according to anyone of claims 1-6, wherein in said aqueous phase said at least one osmotically active electrolytic agent is present in a total concentration comprised between 5.0% and 20% by weight on the total weight of said aqueous phase.

8. Topical composition according to anyone of claims 1-7, wherein the oily phase comprises at least one additional active ingredient selected from depigmenting agents, moisturizing agents, sun filters and/or eutrophic agents, preferably selected among: N-acetylglucosamine, retinyl palmitate, NP ceramide, AP ceramide, EOP ceramide, phytosfingosine, cholesterol, ethylhexyl methoxycinnamate, octocrylene, avobenzone and combinations thereof.

9. Method for preparing the topical composition according to anyone of claims 1 to 8 comprising the following phases:
i. melting in succession at least one of the components A3) selected from cetearyl alcohol, glyceryl stearate, sorbitan stearate, cetearyl glucoside, potassium cetyl phosphate, tocopheryl acetate and oleic acid A1) at 60°C under stirring until dissolution is complete and homogeneous to obtain said oily phase;
ii. dissolving ascorbyl palmitate A2) in said oily phase i) under mechanical stirring until dissolution is complete and homogeneous while maintaining a constant temperature at 60°C;
iii. combining coenzyme Q10 with said oily phase ii) at the same temperature;
iv. cooling to 50°C and adding to this oily phase iii) glutathione in a reduced form until a suspension is obtained;
v. heating the aqueous phase to 50°C and dissolving the osmotically active electrolyte agent, preferably magnesium sulphate anhydrous;
vi. joining the aqueous phase v) to the oily phase iv) at 50°C under stirring for 5 minutes;
vii. cooling to room temperature and adding optionally, at least one of the other active substances referred to in claim 9;
viii. homogenizing the mixture vii).

10. Non therapeutic use of the topical composition according to anyone of claims 1-8, in the cosmetic field.

11. Non therapeutic use of the topical composition according to claim 10, in the treatment and prevention of photo-chrono skin aging and for improving dermal trophism.

12. Non therapeutic use of the topical composition according to claim 10 or 11, wherein the topical composition is applied directly to the skin.

13. Non therapeutic use of the topical composition according to claim 11 or 12, wherein said topical composition, before being applied to the skin, may also be dispersed in oil in water or water in oil emulsions, gels, creams, ointments.

## Patentansprüche

1. Topische Zusammensetzung, umfassend Coenzym Q10 in oxidierter Form und Glutathion in reduzierter Form als Wirkstoffe, umfassend:
A) eine ölige Phase, in der die oben genannten Wirkstoffe dispergiert sind, und umfassend:
A1) Ölsäure,
A2) Ascorbylpalmitat und
A3) mindestens eine der folgenden Komponenten, die die Absorption des Gemischs auf der Hautgewebeebene fördert, ausgewählt aus Cetearylalkohol, Glycerylstearat, Sorbitanstearat, Cetearylglucosid, Kaliumcetylphosphat, Tocopherylacetat;
B) eine wässrige Phase, in der mindestens ein osmotisch aktives Elektrolytmittel, ausgewählt aus Magnesiumsulfat, Magnesiumchlorid, Natriumchlorid, gelöst ist, wobei die ölige Phase A) Ölsäure A1) in Mengen zwischen 20 und 50 Gewichts-%, bezogen auf das Gesamtgewicht der Zusammensetzung, und Ascorbylpalmitat A2) in einem Molverhältnis 1:1 mit der oxidierten Form des Coenzyms Q10 enthält.

2. Topische Zusammensetzung nach Anspruch 1, wobei die ölige Phase A) mindestens zwei, vorzugsweise mindestens drei, besonders bevorzugt mindestens vier Komponenten A3) enthält.

3. Topische Zusammensetzung nach Anspruch 1, wobei die ölige Phase A) mindestens fünf, vorzugsweise alle sechs Komponenten A3) enthält.

4. Topische Zusammensetzung nach einem der Ansprüche 1-3, wobei die ölige Phase A) Coenzym Q10 in Mengen zwischen 0,10 und 10 Gewichts-%, bezogen auf das Gesamtgewicht der Zusammensetzung, und Glutathion in reduzierter Form in Mengen zwischen 0,10 und 10 Gewichts-%, bezogen auf das Gesamtgewicht der Zusammensetzung, enthält.

5. Topische Zusammensetzung nach einem der Ansprüche 1-4, wobei die Konzentration von jeder von mindestens einer der Komponenten A3) zwischen 0,10 und 10 Gewichts-% des Gesamtgewichts der Zusammensetzung liegt und die Gesamtmenge der Komponenten A3) ≤60 Gewichts-% des Gesamtgewichts der Zusammensetzung ist, wenn alle sechs Komponenten A3) vorhanden sind.

6. Formulierung nach einem der Ansprüche 1-5, wobei die wässrige Phase in einer Menge zwischen 5,0 und 10 Gewichts-%, bezogen auf das Gesamtgewicht der Formulierung, vorhanden ist.

7. Topische Zusammensetzung nach einem der Ansprüche 1-6, wobei das mindestens eine osmotisch aktive elektrolytische Mittel in der wässrigen Phase in einer Gesamtkonzentration zwischen 5,0 und 20 Gewichts-%, bezogen auf das Gesamtgewicht der wässrigen Phase, vorhanden ist.

8. Topische Zusammensetzung nach einem der Ansprüche 1-7, wobei die ölige Phase mindestens einen zusätzlichen Wirkstoff umfasst, ausgewählt aus Depigmentierungsmitteln, Befeuchtungsmitteln, Sonnenfiltern und/oder eutrophierenden Mitteln, vorzugsweise ausgewählt aus: N-Acetylglucosamin, Retinylpalmitat, NP-Ceramid, AP-Ceramid, EOP-Ceramid, Phytosfingosin, Cholesterin, Ethylhexylmethoxycinnamat, Octocrylen, Avobenzon und Kombinationen davon.

9. Verfahren zum Herstellen der topischen Zusammensetzung nach einem der Ansprüche 1 bis 8, umfassend die folgenden Phasen:
i. Schmelzen nacheinander mindestens einer der Komponenten A3), ausgewählt aus Cetearylalkohol, Glycerylstearat, Sorbitanstearat, Cetearylglucosid, Kaliumcetylphosphat, Tocopherylacetat und Ölsäure A1) bei 60 °C unter Rühren, bis die Lösung vollständig und homogen ist, um die ölige Phase zu erlangen;
ii. Auflösen von Ascorbylpalmitat A2) in der öligen Phase i) unter mechanischem Rühren, bis die Lösung vollständig und homogen ist, wobei eine konstante Temperatur bei 60 °C beibehalten wird;
iii. Kombinieren von Coenzym Q10 mit der öligen Phase ii) bei der gleichen Temperatur;
iv. Abkühlen auf 50 °C und Hinzufügen von Glutathion in reduzierter Form zu dieser öligen Phase iii), bis eine Suspension erlangt ist;
v. Erwärmen der wässrigen Phase auf 50 °C und Auflösen des osmotisch aktiven Elektrolytmittels, vorzugsweise anhydrisches Magnesiumsulfat;
vi. Verbinden der wässrigen Phase v) mit der öligen Phase iv) bei 50 °C unter Rühren über 5 Minuten;
vii. Abkühlen auf Raumtemperatur und optional Hinzufügen von mindestens einem der anderen in Anspruch 9 genannten Wirkstoffe;
viii. Homogenisieren des Gemischs vii).

10. Nicht-therapeutische Verwendung der topischen Zusammensetzung nach einem der Ansprüche 1-8 auf dem kosmetischen Gebiet.

11. Nicht-therapeutische Verwendung der topischen Zusammensetzung nach Anspruch 10 bei der Behandlung und Prävention von Photochrono-Hautalterung und zum Verbessern von dermalem Trophismus.

12. Nicht-therapeutische Verwendung der topischen Zusammensetzung nach Anspruch 10 oder 11, wobei die topische Zusammensetzung direkt auf die Haut aufgetragen wird.

13. Nicht-therapeutische Verwendung der topischen Zusammensetzung nach Anspruch 11 oder 12, wobei die topische Zusammensetzung vor dem Auftragen auf die Haut auch in Öl in Wasser oder Wasser in Ölemulsionen, Gelen, Cremes, Salben dispergiert werden kann.

## Revendications

1. Composition topique comprenant la coenzyme Q10 sous forme oxydée et le glutathion sous forme réduite en tant qu'ingrédients actifs comprenant :
A) une phase huileuse dans laquelle les ingrédients actifs susmentionnés sont dispersés et comprenant :
A1) l'acide oléique,
A2) le palmitate d'ascorbyle et
A3) au moins l'un des composants suivants favorisant l'absorption du mélange au niveau des tissus cutanés sélectionnés parmi l'alcool cétéarylique, le stéarate de glycéryle, le stéarate de sorbitan, le glucoside de cétéaryle, le phosphate cétylique de potassium, l'acétate de tocophéryle ;
B) une phase aqueuse dans laquelle au moins un agent électrolytique osmotiquement actif, choisi parmi le sulfate de magnésium, le chlorure de magnésium, le chlorure de sodium, est dissous,
dans laquelle la phase huileuse A) contient de l'acide oléique A1) en quantités comprises entre 20 % et 50 % en poids par rapport au poids total de la composition et du palmitate d'ascorbyle A2) dans un rapport molaire 1:1 avec la forme oxydée de la coenzyme Q10.

2. Composition topique selon la revendication 1, dans laquelle la phase huileuse A) contient au moins deux, de préférence au moins trois, plus préférablement au moins quatre composants A3).

3. Composition topique selon la revendication 1, dans laquelle la phase huileuse A) contient au moins cinq, de préférence tous les six composants A3).

4. Composition topique selon l'une quelconque des revendications 1 à 3, dans laquelle la phase huileuse A) contient de la coenzyme Q10 en quantités comprises entre 0,10 % et 10 % en poids par rapport au poids total de la composition et du glutathion sous forme réduite en quantités comprises entre 0,10 % et 10 % en poids par rapport au poids total de la composition.

5. Composition topique selon l'une quelconque des revendications 1 à 4, dans laquelle la concentration de chacun d'au moins un des composants A3) est comprise entre 0,10 % et 10 % en poids du poids total de la composition et la quantité totale desdits composants A3) est ≤60 % en poids par rapport au poids total de la composition, lorsque les six composants A3) sont présents.

6. Composition topique selon l'une quelconque des revendications 1 ou 5, dans laquelle la phase aqueuse est présente en une quantité comprise entre 5,0% et 10% en poids par rapport au poids total de la composition.

7. Composition topique selon l'une quelconque des revendications 1 à 6, dans laquelle, dans ladite phase aqueuse, ledit au moins un agent électrolytique osmotiquement actif est présent à une concentration totale comprise entre 5,0 % et 20 % en poids par rapport au poids total de ladite phase aqueuse.

8. Composition topique selon l'une quelconque des revendications 1 à 7, dans laquelle la phase huileuse comprend au moins un ingrédient actif supplémentaire choisi parmi les agents dépigmentants, les agents hydratants, les filtres solaires et/ou les agents eutrophes, de préférence choisi parmi : N-acétylglucosamine, palmitate de rétinyle, céramide NP, céramide AP, céramide EOP, phytosfingosine, cholestérol, méthoxycinnamate d'éthylhexyle, octocrylène, avobenzone et leurs combinaisons.

9. Procédé de préparation de la composition topique selon l'une quelconque des revendications 1 à 8, comprenant les étapes suivantes :
i. la fusion successive d'au moins un des composants A3) choisi parmi l'alcool cétéarylique, le stéarate de glycéryle, le stéarate de sorbitan, le glucoside de cétéaryle, le phosphate cétylique de potassium, l'acétate de tocophéryle et l'acide oléique A1) à 60°C sous agitation jusqu'à dissolution complète et homogène pour obtenir ladite phase huileuse ;
ii. la dissolution du palmitate d'ascorbyle A2) dans ladite phase huileuse i) sous agitation mécanique jusqu'à ce que la dissolution soit complète et homogène tout en maintenant une température constante à 60°C ;
iii. la combinaison de la coenzyme Q10 avec ladite phase huileuse ii) à la même température ;
iv. le refroidissement à 50°C et l'ajout à cette phase huileuse iii) du glutathion sous forme réduite jusqu'à l'obtention d'une suspension ;
v. le chauffage de la phase aqueuse à 50°C et la dissolution de l'agent électrolytique osmotiquement actif, de préférence le sulfate de magnésium anhydre ;
vi. l'assemblage de la phase aqueuse v) à la phase huileuse iv) à 50°C sous agitation pendant 5 minutes ;
vii. le refroidissement à température ambiante et l'ajout éventuel d'au moins une des autres substances actives visées à la revendication 9 ;
viii. l'homogénéisation du mélange vii).

10. Utilisation non thérapeutique de la composition topique selon l'une quelconque des revendications 1 à 8, dans le domaine cosmétique.

11. Utilisation non thérapeutique de la composition topique selon la revendication 10, dans le traitement et la prévention du vieillissement cutané photochronologique et pour améliorer le trophisme dermique.

12. Utilisation non thérapeutique de la composition topique selon la revendication 10 ou 11, dans laquelle la composition topique est appliquée directement sur la peau.

13. Utilisation non thérapeutique de la composition topique selon la revendication 11 ou 12, dans laquelle ladite composition topique, avant d'être appliquée sur la peau, peut également être dispersée dans de l'huile dans l'eau ou de l'eau dans des émulsions d'huile, des gels, des crèmes, des onguents.
